# EUROPEAN PATENT APPLICATION

(11) **EP 4 438 725 A1**
(43) Date of publication of application: **02.10.2024**
(21) Application number: 23305474.1
(22) Date of filing: 31.03.2023
(51) Int. Cl.: C12N 9/18, C08J 11/10, D06M 16/00, C12N 9/52

(54) **NOVEL PROTEASES AND USES THEREOF**

(71) Applicant: Carbios, 63100 Clermont-Ferrand (FR)
(72) Inventor: MARTY, Alain, 63100 Clermont-Ferrand (FR); GUICHERD, Marie, 63100 Clermont-Ferrand (FR); GUEROULT, Marc, 63100 Clermont-Ferrand (FR); DOQUESNE, Sophie, 31024 Toulouse (FR); ANDRE, Isabelle, 31077 Toulouse (FR)
(74) Representative: Cabinet Becker et Associés

(57) **Abstract**

The present invention relates to novel proteases, more particularly to protease variants having improved activity compared to the protease of SEQ ID N°1 and the uses thereof for degrading polyester containing material, such as plastic products. The proteases of the invention are particularly suited to degrade polylactic acid, and material containing polylactic acid.

## Description

The present invention relates to novel proteases, more particularly to proteases having improved activity compared to a parent protease and uses thereof for degrading polyester or polyester containing material, such as plastic products. The proteases of the invention are particularly suited to degrade polylactic acid and polylactic acid containing material.

### BACKGROUND

Proteases are able to catalyze the hydrolysis of a variety of polymers, including polyesters. In this context, proteases have shown promising effects in a number of industrial applications, including as detergents for dishwashing and laundry applications, as degrading enzymes for processing biomass and food, as biocatalysts in the detoxification of environmental pollutants or for the treatment of polyester fabrics in the textile industry. Likewise, the use of proteases as degrading enzymes for hydrolyzing polylactic acid (PLA) is of particular interest. Indeed, PLA is a bio-based polymer (i.e., a polymer derived from natural and/or renewable sources) that is used in a large number of technical fields, such as flexible and rigid packaging, bags, mulching films, as well as in the manufacture of clothes and carpets. Accordingly, PLA accumulation in landfills becomes an increasing ecological problem.

Among proteases, serine proteases (EC 3.4.21) are enzymes that cleave peptide amide bonds in proteins, in which serine serves as the nucleophilic amino acid in the enzyme active site. Serine proteases are found ubiquitously in both eukaryotes and prokaryotes. Numerous bacterial serine proteases have been identified initially in *Bacillus* and more recently in other mesophilic hosts. However, an increasing number of serine proteases have been isolated from thermophilic and hyperthermophilic bacteria.

Biological degradation, and more particularly enzymatic degradation, is considered as an interesting solution to decrease plastic waste accumulation. Indeed, enzymes are able to accelerate hydrolysis of polyester containing material, and more particularly of plastic products, even down to the monomer level. Furthermore, the hydrolysate (i.e., monomers and oligomers) can be recycled as material for the synthesis of new polymers. Recently, new plastic materials have been developed that integrate biological entities suitable for degrading at least one polymer of the plastic material, leading to the production of biodegradable plastic products. As an example, plastic products made of PLA and including proteases have been produced. Such biodegradable plastics may at least partially solve the problem of plastic build-up in landfill sites and natural habitats.

In this context, several proteases have been identified as candidate degrading enzymes. For instance, a protease of *Actinomadura sp.* (as described in WO 2016/062695) and variants thereof (as described in WO 2019/122308, WO 2021/099337 and WO 2022/144247) exhibit capacity to degrade polyester, and more particularly polylactic acid.

However, there is still a need for proteases with improved activity to allow a degrading process with higher efficiency, and thereby enhancing the competitiveness of biodegradable plastic production processes, biological polyester degrading processes and/or biological recycling processes.

### SUMMARY OF THE INVENTION

The present invention provides new variants of proteases exhibiting increased polyester degrading activity compared to their parent protease. These proteases are particularly useful in processes for degrading polyester(s) and/or plastic material and within plastic material and products containing polyester(s), such as polylactic acid (PLA). More particularly, the present invention provides variants of a protease having the amino acid sequence as set forth in SEQ ID N°1, referenced herein as the parent protease. The present invention further provides processes for degrading polyester(s) and/or plastic material and product containing polyester(s), and more particularly polylactic acid (PLA) and/or plastic material and product containing PLA, using a variant of the invention.

It is an object of the invention to provide a protease variant which comprises an amino acid sequence which (i) has at least 90%, 95%, 96%, 97%, 98% or 99% identity to the full length amino acid sequence set forth in SEQ ID N°1, and (ii) has at least one amino acid substitution selected from the group consisting in S101A/V/G/H/I/K/R/T/N/Q, G102A, S103A/F/I/V/W/H/K/M/Q/R/T/N/Y, T106V, G131L/T/V and I217L wherein the positions are numbered by reference to the amino acid sequence set forth in SEQ ID N°1, (iii) has a polyester degrading activity, and (iv) exhibits increased polyester degrading activity compared to the protease of SEQ ID N°1.

Preferably, the protease comprises at least one substitution selected from S101A/V, G102A, S103A/F/I/V/W and G131L/T, preferably selected from S101V, S103A/F/I/V/W and G131T, more preferably selected from S103F/I/W even more preferably at least the substitution S103F.

It is another object of the invention to provide a nucleic acid encoding a protease as defined above. The present invention also relates to an expression cassette or an expression vector comprising said nucleic acid, and to a host cell comprising said nucleic acid, expression cassette or vector.

It is a further object of the invention to provide a method of producing a protease as defined above comprising:
(a) culturing a host cell as defined above under conditions suitable to express a nucleic acid encoding a protease; and optionally
(b) recovering said protease from the cell culture.

The present invention also relates to a method of degrading a plastic product containing at least one polyester, preferably at least PLA, comprising
(a) contacting the plastic product with a protease or host cell as defined above, thereby degrading the plastic product; and optionally
(b) recovering monomers and/or oligomers, preferably monomers and/or oligomers of lactic acid (LA).

The present invention also relates to a polyester containing material comprising a protease or host cell according to the invention. The present invention relates more preferably to a polylactic acid (PLA) containing material comprising a protease or host cell or composition according to the invention able to degrade said PLA. The invention also provides a process for producing such polyester containing material comprising a step of mixing a polyester, preferably PLA, and a protease or host cell or composition according to the invention, preferably able to degrade PLA, wherein the mixing step is performed at a temperature at which the polyester is in a partially or totally molten state, preferably during an extrusion process.

The present invention further relates to the use of a protease as described above for degrading a polyester containing material, more preferably a PLA containing material.

### DETAILED DESCRIPTION OF THE INVENTION

### Definitions

The present disclosure will be best understood by reference to the following definitions.

Herein, the terms *"peptide"*, *"polypeptide"*, *"protein"*, *"enzyme"* refer to a chain of amino acids linked by peptide bonds, regardless of the number of amino acids forming said chain. The amino acids are herein represented by their one-letter or three-letters code according to the following nomenclature: A: alanine (Ala); C: cysteine (Cys); D: aspartic acid (Asp); E: glutamic acid (Glu); F: phenylalanine (Phe); G: glycine (Gly); H: histidine (His); I: isoleucine (Ile); K: lysine (Lys); L: leucine (Leu); M: methionine (Met); N: asparagine (Asn); P: proline (Pro); Q: glutamine (Gln); R: arginine (Arg); S: serine (Ser); T: threonine (Thr); V: valine (Val); W: tryptophan (Trp ) and Y: tyrosine (Tyr).

The term *"protease"* refers to an enzyme which belongs to a class of hydrolases classified as EC 3.4 according to Enzyme Nomenclature that is able to catalyze the hydrolysis of a peptide bond in a peptide or a protein. The term *"serine protease"* refers to proteases classified as EC 3.4.21 according to the nomenclature of the Enzyme Commission.
The term *"parent protein"* refers to the protease having the amino acid sequence as set forth in SEQ ID N°1.

The terms "*mutant*" and *"variant"* may be used interchangeably to refer to polypeptides derived from a parent polypeptide and comprising at least one modification or alteration, i.e., a substitution, insertion, and/or deletion, at one or more positions. Variants may be obtained by various techniques well known in the art. In particular, examples of techniques for altering a DNA sequence encoding the parent protein, include, but are not limited to, site-directed mutagenesis, random mutagenesis and synthetic oligonucleotide construction. Thus, the terms *"modification"* and *"alteration"* as used herein, in relation to a particular position, means that at least the amino acid in this particular position has been modified compared to the amino acid in this particular position in the parent protein.

A *"substitution"* means that an amino acid residue is replaced by another amino acid residue. Preferably, the term "substitution" refers to the replacement of an amino acid residue by another selected from the naturally-occurring standard 20 amino acid residues, rare naturally occurring amino acid residues (e.g. hydroxyproline, hydroxylysine, allohydroxylysine, 6-N-methylysine, N-ethylglycine, N-methylglycine, N-ethylasparagine, allo-isoleucine, N-methylisoleucine, N-methylvaline, pyroglutamine, aminobutyric acid, ornithine, norleucine, norvaline), and non-naturally occurring amino acid residue, often made synthetically, (e.g. cyclohexyl-alanine). Preferably, the term *"substitution"* refers to the replacement of an amino acid residue by another selected from the naturally-occurring standard 20 amino acid residues (G, P, A, V, L, I, M, C, F, Y, W, H, K, R, Q, N, E, D, S and T). The sign "+" indicates a combination of substitutions. In the present document, the following terminology is used to designate a substitution: Y167R denotes that amino acid residue Tyrosine (Y) at position 167 of a parent sequence is substituted by an Arginine (R). Y167V/I/M denotes that amino acid residue Tyrosine (Y) at position 167 of a parent sequence is substituted by one of the following amino acids: Valine (V), Isoleucine (I), or Methionine (M). The substitution can be a conservative or non-conservative substitution. Examples of conservative substitutions are within the groups of basic amino acids (arginine, lysine and histidine), acidic amino acids (glutamic acid and aspartic acid), polar amino acids (glutamine, asparagine and threonine), hydrophobic amino acids (methionine, leucine, isoleucine, cysteine and valine), aromatic amino acids (phenylalanine, tryptophan and tyrosine), and small amino acids (glycine, alanine and serine).

Unless otherwise specified, the positions disclosed in the present application are numbered by reference to the amino acid sequence set forth in SEQ ID N°1.

As used herein, the term *"sequence identity"* or *"identity"* refers to the number (or fraction expressed as a percentage %) of matches (identical amino acid residues) between two polypeptide sequences. The sequence identity is determined by comparing the sequences when aligned so as to maximize overlap and identity while minimizing sequence gaps. In particular, sequence identity may be determined using any of a number of mathematical global or local alignment algorithms, depending on the length of the two sequences. Sequences of similar lengths are preferably aligned using a global alignment algorithm (e.g. Needleman and Wunsch algorithm; Needleman and Wunsch, 1970) which aligns the sequences optimally over the entire length, while sequences of substantially different lengths are preferably aligned using a local alignment algorithm (e.g. Smith and Waterman algorithm (Smith and Waterman, 1981) or Altschul algorithm (Altschul et al., 1997; Altschul et al., 2005)). Alignment for purposes of determining percent amino acid sequence identity can be achieved in various ways that are within the skill in the art, for instance, using publicly available computer software available on internet web sites such as http://blast.ncbi.nlm.nih.gov/ or http://www.ebi.ac.uk/Tools/emboss/). Those skilled in the art can determine appropriate parameters for measuring alignment, including any algorithms needed to achieve maximal alignment over the full length of the sequences being compared. For purposes herein, % amino acid sequence identity values refers to values generated using the pair wise sequence alignment program EMBOSS Needle that creates an optimal global alignment of two sequences using the Needleman-Wunsch algorithm, wherein all search parameters are set to default values, i.e. Scoring matrix = BLOSUM62, Gap open = 10, Gap extend = 0.5, End gap penalty = false, End gap open = 10 and End gap extend = 0.5. The percentage of identity is calculated by determining the number of identical positions between these two sequences, by dividing this number by the total number of compared positions, and by multiplying the result obtained by 100 to get the percentage of identity between these two sequences.

The term *"recombinant"* refers to a nucleic acid construct, a vector, a polypeptide or a cell produced by genetic engineering.

The term *"expression",* as used herein, refers to any step involved in the production of a polypeptide such as transcription, post-transcriptional modification, translation, post-translational modification, or secretion.

According to the invention, *"oligomers"* refer to molecules containing from 2 to about 20 monomers.

In the present description, *"polyesters"* encompass polylactic acid (PLA), polyethylene terephthalate (PET), polytrimethylene terephthalate (PTT), polybutylene terephthalate (PBT), polyethylene isosorbide terephthalate (PEIT), polyhydroxyalkanoate (PHA), polybutylene succinate (PBS), polybutylene succinate adipate (PBSA), polybutylene adipate terephthalate (PBAT), polyethylene furanoate (PEF), polycaprolactone (PCL), or polyethylene adipate) (PEA), as well as any blends/mixtures of these polymers. In a particular embodiment, "polyester" also encompasses poly(glycolic acid) (PGA) or poly(lactic-co-glycolic acid) (PLGA) as well as any blends/mixtures of these polymers.

In the context of the invention, a *"polyester containing material*" or *"polyester containing product*" refers to a product, such as plastic product, comprising at least one polyester in crystalline, semi-crystalline or totally amorphous form. In a particular embodiment, the polyester containing material refers to any item made from at least one plastic material, such as plastic sheet, tube, rod, profile, shape, film, massive block, fiber, textiles, etc., which contains at least one polyester, and possibly other substances or additives, such as plasticizers, mineral or organic fillers. In another particular embodiment, the polyester containing material refers to textile, fabrics or fibers comprising at least one polyester. In another particular embodiment, the polyester containing material refers to plastic waste or fiber waste comprising at least one polyester. In another particular embodiment, the polyester containing material refers to a plastic compound, or plastic formulation, in a molten or solid state, suitable for making a plastic product.

Within the context of the invention, the term *"increased degrading activity"* indicates an increased ability of the enzyme to degrade a polyester, preferably PLA, or a plastic product or material, comprising at least a polyester, preferably at least PLA, as compared to the protease of SEQ ID N°1. Such an increase is typically of about 10%, 20%, 30%, 40%, 50%, 100%, 200%, 300%, 400%, 500% or more in comparison to the parent protease.

The activity of a protein may be evaluated by the one skilled in the art, according to methods known per se in the art. For instance, the activity can be assessed by the measurement of the specific protease activity rate, the measurement of the hydrolysis of pNA (N-succinyl-Ala-Ala-Ala-p-nitroanilide), the measurement of the specific polyester's depolymerization activity rate, the measurement of the rate to degrade a solid polyester compound or protein dispersed in an agar plate, the measurement of the decrease of the turbidity of an emulsion containing a polyester, or the measurement of the specific polyester's depolymerization activity rate in reactor.

Within the context of the invention, the term *"specific degrading activity"* for a targeted polyester designates the initial rate of monomers and/or oligomers, in mg, released per hour and per mg of enzyme under suitable conditions of temperature, pH and buffer, when contacting a target polyester or a plastic product containing said target polyester with a protease according to the invention. As an example, the specific degrading activity for PLA corresponds to the mg of lactic acid and dimers of lactic acid produced per hour and per mg of enzyme, or to the µmol of PLA hydrolyzed/min and per mg of enzyme, as determined in the linear part of the hydrolysis curve, i.e., the part starting at time zero that is tangent to the curve.

### Novel proteases

By working on development of novel proteases having improved polyester-degrading activity as compared to enzymes currently available, the inventors have further worked on the serine protease having the amino acid sequence of SEQ ID N°1 and have been able to develop variants thereof that exhibit improved polyester-degrading activity. In particular, the inventors have identified specific amino acid residues within the protein, which are intended to be in contact with a polyester substrate and which may be advantageously modified to promote the contact of the polyester substrate with the protein. Without being bound by theory, it is believed such modifications increase adsorption of the protease on the polyester resulting in an improved degrading activity. The inventors have thus been able to develop novel proteases derived from SEQ ID N°1 that show higher activity and are particularly suited to degrade polyester or polyester-containing products. Interestingly, these new proteases have superior properties for use in industrial processes. The proteases newly developed exhibit an improved activity at least in conditions at which industrial production of degradable plastic products can be performed and/or environmental degradation of plastic products can be obtained. Interestingly, the thermostability of such new proteases is not impaired or not significatively impaired as compared to the thermostability of the parent protease.

SEQ ID N°1:







The present disclosure relates to a protease which comprises an amino acid sequence which (i) has at least 75%, 80%, 85%, 90%, 95%, 96%, 97%, 98% or 99% identity to the full length amino acid sequence set forth in SEQ ID N°1, and (ii) has at least one amino acid substitution selected from the group consisting in S103F/A/I/VAV/H/K/M/Q/R/T, S101A/V/G/H/I/K/R/T, G102A, T106V, G131L/T/V and I217L, wherein the positions are numbered by reference to the amino acid sequence set forth in SEQ ID N°1, (iii) has a polyester degrading activity, and (iv) exhibits increased polyester degrading activity compared to the protease of SEQ ID N°1.

Said protease may comprise at least one substitution selected from S101A/V, G102A, S103A/F/I/V/W and G131L/T, preferably selected from S101V, S103A/F/I/V/W and G131T, more preferably selected from S103F/I/W even more preferably at least the substitution S103F.

The present disclosure also relates to a protease which comprises an amino acid sequence which (i) has at least 85%, 90%, 95%, 96%, 97%, 98% or 99% identity to the full length amino acid sequence set forth in SEQ ID N°1, and (ii) has at least one amino acid substitution selected from the group consisting in S101A/V/G/H/I/K/R/T/N/Q, G102A, S103A/F/I/V/W/H/K/M/Q/R/T/N, T106V, G131L/T/V and I217L wherein the positions are numbered by reference to the amino acid sequence set forth in SEQ ID N°1, (iii) has a polyester degrading activity, and (iv) exhibits increased polyester degrading activity compared to the protease of SEQ ID N°1.

It is an object of the invention to provide a protease which is a variant of the protease of SEQ ID N°1, and which comprises an amino acid sequence which has at least 90%, 95%, 96%, 97%, 98% or 99% identity to the full length amino acid sequence set forth in SEQ ID N°1, and (ii) has at least one amino acid substitution selected from the group consisting in S101A/V/G/H/I/K/R/T/N/Q, G102A, S103A/F/I/V/W/H/K/M/Q/R/T/N/Y, T106V, G131L/T/V and I217L wherein the positions are numbered by reference to the amino acid sequence set forth in SEQ ID N°1, (iii) has a polyester degrading activity, and (iv) exhibits increased polyester degrading activity compared to the protease of SEQ ID N°1.

In particular, the protease may comprise at least one substitution selected from S101A/V, G102A, S 103A/F/I/V/W and G131L/T, preferably selected from S101V, S103A/F/I/V/W and G131T, more preferably selected from S103F/I/W even more preferably at least the substitution S103F

In a particular embodiment, the protease variant comprises a single substitution as compared to SEQ ID N°1, selected from the group consisting in S101A/V/G/H/I/K/R/T/N/Q, G102A, S103A/F/I/V/W/H/K/M/Q/R/T/N/Y, T106V, G131L/T/V and I217L. In particular, the protease variant comprises a single substitution selected from S101A/V, G102A, S103A/F/I/V/W and G131L/T, preferably selected from S101V, S103A/F/I/V/W and G131T, more preferably selected from S103F/I/W, even more preferably at least the substitution S103F.

According to a particular embodiment of the present invention, the protease has the amino acid sequence as set forth in SEQ ID N°1, except the substitution S101A (V1), S101G (V2), S101H (V3), S101I (V4), S101K (V5), S101N (V6), S101Q (V7), S101R (V8), S101T (V9), S101V (V10), S103A (V11), S103F (V12), S103H (V13), S103I (V14), S103K (V15), S103M (V16), S103N (V17), S103Q (V18), S103R(V19), S103T (V20), S103V (V21), S103W (V22), S103Y (V23), T106V (V24), G131L (V26), G131T (V26), G131V (V27), I217L (V28) or G102A (V29).

In another particular embodiment, the protease variant comprises two or more substitutions as compared to SEQ ID N°1, wherein at least one substitution is selected from the group consisting in S101A/V/G/H/I/K/R/T/N/Q, G102A, S103A/F/I/V/W/H/K/M/Q/R/T/N/Y, T106V, G131L/T/V and I217L, preferably at least one substitution selected from S101A/V, G102A, S103A/F/I/V/W and G131L/T, more preferably selected from S101V, S103A/F/I/V/W and G131T, even more preferably selected from S103F/I/W, even more preferably the substitution S103F.

In another particular embodiment, the protease variant comprises at least two substitutions selected from the group consisting in S101A/V/G/H/I/K/R/T/N/Q, G102A, S103A/F/I/V/W/H/K/M/Q/R/T/N/Y, T106V, G131L/T/V and I217L, preferably at least two substitutions selected from the group consisting in S101A/V, G102A, S103A/F/I/V/W and G131L/T.

In addition to any of the above embodiments, the protease may further comprise at least one substitution at a position selected from L138, N139, N143, T160, R166, S170, N173, T175, S194, H197, L210, G212, 1217, R247, G133, D12, L21, N4, S8, L11, D12, D15, Q16, S22, S24, Y25, T30, G31, V34, N35, T41, T45, F50, A54, N69, G72, T78, G80, A83, A87, K88, A89, V90, L92, A117, P123, A124, N127, S135, S137, S148, A153, N158, T160, N162, A163, S167, A169, A172, A174, T175, T176, A179, R186, A187, Y189, N191, G193, H197, G202, S203, S204, T206, L210, G213, S218, A223, P225, A228, G229, T230, A232, K235, G239, S244, I246, G261, K273, S275, W9, T17, G31, A32, G33, V34, N35, A36, Y37, I39, I43, Y44, A46, G52, T55, N56, A60, N64, A76, A87, V90, R93, V95, G108, A111, G112, V116, A117, V121, V125, M128, A142, N143, L145, A146, V150, L152, A153, V154, C164, A169, A171, A172, N173, A174, T175, T176, A179, S182, R186, A187, S194, H197, S203, L210, N211, G212, G213, V227, T230, A236, F243, R247, N253, N262, S264, T266 and N272. Preferably, the protease further comprises at least one substitution selected from L138A, N139D, N143R, T160A, R166K, S170R, N173E, T175C, S194P, H197D, L210P, G212N, I217K, R247C, G133K, D12C, L21C, N4T, S8C, L11C, D12C, D15C, Q16C, S22C, S24C, Y25C, T30C, G31C, V34C, N35C, T41C, T45C, F50C, A54C, N69C, G72C, T78C, G80C, A83T, A87C, K88C, A89C, V90C, L92C, A117C, P123C, A124C, N127C, S135C, S137A, S148C, A153C, N158C, T160N, N162C, A163C, S167C, A169C, A172C, A174C, T175C, T176C, A179C, R186C, A187C, Y189C, N191C, G193C, H197C, G202C, S203C, S204D, T206C, L210C, G213C, S218C, A223C, P225C, A228C, G229C, T230V, A232C, K235L, G239P, S244A, I246V, G261C, K273T, S275G, W9C, T17E/D, G31C, A32E/D, G33C, V34C, N35C, A36C, Y37C, I39C, I43C, Y44A, A46W, G52D/E, T55C, N56C, A60C, N64D/E, A76C, A87C, V90C, R93C/H, V95C, G108A, A111C, G112C, V116C, A117C, V121C, V125C, M128C, A142C, N143E/D, L145C, A146C, V150C, L152C, A153C, V154C, C164A, A169C, A171C, A172C, N173C/H, A174C, T175C, T176C, A179C, S182C/D/E, R186C, A187C, S194E/D, H197D/E, S203C, L210S/T/Y, N211E/D/T/S, G212E/D/S/T, G213A/E/D/S/T, V227C, T230A/V, A236C, F243C, R247C, N253C, N262C, S264P, T266C and N272C.

In a particular embodiment, the protease comprises at least one amino acid substitution selected from the group consisting in S101A/V/G/H/I/K/R/T/N/Q, G102A, S103A/F/I/V/W/H/K/M/Q/R/T/N/Y, T106V, G131L/T/V and I217L and further comprises the substitution G133K. Preferably, the protease comprises at least one amino acid substitution selected from the group consisting in S101A/V, G102A, S103A/F/I/V/W and G131L/T and further comprises the substitution G 133K.

In a particular embodiment, the protease comprises at least one amino acid substitution selected from the group consisting in S103A/F/I/V/W/H/K/M/Q/R/T/N/Y, G102A, T106V, G131L/T/V and I217L and one additional substitution selected from S101F/L/M/W/Y, more preferably S101F. Preferably, the protease comprises at least one amino acid substitution selected from the group consisting in G102A, S103A/F/I/V/W and G131L/T and one additional substitution selected from S101F/L/M/W/Y, more preferably S101F. More preferably, the protease comprises at least one amino acid substitution selected S103F/I/W even more preferably at least the substitution S103F and one additional substitution selected from S101F/L/M/W/Y, more preferably S101F. In a particular embodiment, the protease comprises at least the combination S103F + S101F.

In another particular embodiment, the protease comprises at least one amino acid substitution selected from the group consisting in S101A/V/G/H/I/K/R/T/N/Q, G102A, T106V, G131L/T/V and I217L and the additional substitution S103L. Preferably, the protease comprises at least one amino acid substitution selected from the group consisting in S101A/V, G102A, and G131L/T and the additional substitution S103L.

In another particular embodiment, the protease comprises at least one amino acid substitution selected from the group consisting in S101A/V/G/H/I/K/R/T/N/Q, G102A, S103A/F/I/V/W/H/K/M/Q/R/T/N/Y, G131L/T/V and I217L and one additional substitution selected from T106I/L. Preferably, the protease comprises at least one amino acid substitution selected from the group consisting in S101A/V, G102A, S103A/F/I/V/W and G131L/T and one additional substitution selected from T106I/L. More preferably, the protease comprises at least one amino acid substitution selected S103F/I/W even more preferably at least the substitution S103F and one additional substitution selected from T106I/L. In a particular embodiment, the protease comprises at least the combination S103F + T106I/L.

In another particular embodiment, the protease comprises at least one amino acid substitution selected from the group consisting in S101A/V/G/H/I/K/R/T/N/Q, G102A, S103A/F/I/V/W/H/K/M/Q/R/T/N/Y, T106V and I217L and the additional substitution G131I. Preferably, the protease comprises at least one amino acid substitution selected from the group consisting in S101A/V, G102A and S103A/F/I/V/W and the additional substitution G131I.

In a particular embodiment, the protease comprises at least one amino acid substitution selected from the group consisting in S103A/F/I/V/W/H/K/M/Q/R/T/N/Y, G102A, G131L/T/V and I217L, preferably selected from G102A, S103A/F/I/V/W and G131L/T, more preferably selected from S103F/I/W and further comprises at least one substitution selected from S101F/L/M/W/Y and T106I/L, preferably at least one substitution selected from S101F and T106I/L. In a preferred embodiment, the protease comprises at least one amino acid substitution selected from the group consisting in S 103A/F/I/V/W/H/K/M/Q/R/T/N/Y, G102A, G131L/T/V and I217L and further comprises a combination of substitutions selected from S101F/L/M/W/Y + T106I/L, preferably selected from S101F + T106I/L.

In an embodiment the protease comprises at least one amino acid substitution selected from the group consisting in S103A/F/I/V/W, G102A and G131L/T and further comprises at least one substitution selected from S101F/L/M/W/Y and T106I/L, preferably at least one substitution selected from S101F and T106I/L. More preferably, the protease comprises at least one amino acid substitution selected S103F/I/W, even more preferably at least the substitution S103F and further comprises at least one substitution selected from S101F/L/M/W/Y and T106I/L, preferably at least one substitution selected from S101F and T106I/L.

In an embodiment, the protease comprises at least one amino acid substitution selected from the group consisting in S103A/F/I/V/W, G102A and G131L/T and further comprises a combination of substitutions selected from S101F/L/M/W/Y + T106I/L, even more preferably selected from S101F + T106I/L. In particular, the protease comprises at least one amino acid substitution selected S103F/I/W, even more preferably at least the substitution S103F and further comprises a combination of substitution selected from S101F/L/M/W/Y + T106I/L, even more preferably selected from S101F + T106I/L. In a particular embodiment, the protease comprises at least a combination of substitutions selected from S101F + S103F + T106I and S101F + S103F + T106L.

In a particular embodiment, the protease has the amino acid sequence as set forth in SEQ ID N°1, except the combination of substitutions S101F + S103F + T106I (V30) or the combination S101F + S103F + T106L (V31).

In an embodiment, the protease comprises at least one amino acid substitution selected from the group consisting in G102A, G131L/T/V and I217L and further comprises at least a combination of substitutions selected from S101F/L/M/W/Y + S103L + T106I/L, preferably selected from S101F + S103L + T106I and S101F + S103L + T106L.

In an embodiment, the protease of the invention comprises at least one amino acid substitution selected from the group consisting in G102A, G131L/T/V and I217L and further comprises at least a combination of substitutions selected from S101F + S103F + T106I and S101F + S103F + T106L.

In an embodiment, the protease comprises at least one amino acid residue selected from D40, H71, S221, C68, C100, C164, C195, V75, L98, G104, T105, V109, N127, L130, G132, G133, S135, L138, A155, G157, E159, R166, S218, M222, A223, T224, P225 as in the parent protease, i.e., the protease of the invention is not modified at one, two, three, etc., or all of these positions. Preferably, the protease comprises at least one amino acid residue selected from D40, H71, S221, C68, C100, C164 or C195 as in the parent protease.

In a preferred embodiment, any protease of the invention comprises at least one amino acid residue selected from D40, H71 and S221 forming the catalytic site, as in the parent protease, i.e. the protease of the invention is not modified at one, two or all of these positions. Preferably, the protease comprises the combination D40 + H71 + S221, as in the parent protease.

Alternatively or in addition, any protease of the invention comprises at least one amino acid residue selected from C68, C100, C164 or C195 forming disulfide bonds, as in the parent protease, i.e. the protease of the invention is not modified at one, two or more of these positions. Preferably, the protease comprises the combination C68 + C100, C164 + C195 or C68 + C100 + C164 + C195 as in the parent protease.

In an embodiment, any protease of the invention comprises at least one combination selected from D40 + H71 + S221 + C68 + C100, D40 + H71 + S221 + C164 + C195 and D40 + H71 + S221 + C68 + C100 + C164 + C195 as in the parent protease.

Alternatively or in addition, any protease of the invention comprises at least one amino acid residue selected from V75, L98, G104, T105, V109, N127, L130, G132, G133, S135, L138, A155, G157, E159, R166, S218, M222, A223, T224, or P225 which correspond to residues in contact with the polyester (e.g., PLA) during the degradation reaction, as in the parent protease, i.e. the protease of the invention is not modified at one, two or more of these positions.

### Propeptide

Advantageously, the protease variant comprises, at the N-terminal end of the amino acid sequence having % identity with SEQ ID N°1, an amino acid sequence acting as a *"propeptide"* which is involved in the 3D folding and the maturation of the protease.

Particularly, the protease variant may comprise a propeptide sequence, which has at least 75%, 80%, 85%, 90%, 95%, 96%, 97%, 98%, 99% or 100% identity to the full length amino acid sequence set forth in SEQ ID N°2. In a particular embodiment, the protease variant of the invention comprises at the N-terminal end a propeptide sequence having the amino acid sequence set forth in SEQ ID N°2.

SEQ ID N°2:

APAVPVAMAAAGQGVAGQYIVTLKKGVSVDSTVAKRGIRTQHRFGKVLNGFSAKLT
DDQLSKLRTTPGVASIEQDAVITVD

### Polyester degrading activity

It is an object of the invention to provide new enzymes having a polyester degrading activity, preferably a polylactic acid degrading activity. Preferably, the protease of the invention exhibits an increased polyester degrading activity compared to the protease of SEQ ID N°1. In particular, the protease of the invention exhibits an increased PLA degrading activity compared to the protease of SEQ ID N°1.

Advantageously, the protease variant of the invention exhibits a polyester degrading activity at least in a range of temperatures from 10°C to 90°C, preferably from 20°C to 70°C, more preferably from 30°C to 60°C, even more preferably at 45°C +/-5°C.

In another embodiment, the protease variant exhibits a polyester degrading activity at 70°C +/-5°C. In a particular embodiment, a polyester degrading activity is still measurable at a temperature between 40°C and 60°C. In another particular embodiment, the polyester degrading activity is still measurable at a temperature between 10°C and 30°C, such as between 20°C and 30°C, preferably between 15°C and 28°C, in particular at 28°C +/-2°C, corresponding to the mean temperature in the natural environment.

In a particular embodiment, the protease variant of the invention has an increased polyester degrading activity at a given temperature, compared to the protease of SEQ ID N°1, and more particularly at a temperature between 20°C and 80°C, more preferably between 30°C and 70°C, even more preferably between 40°C and 60°C, even more preferably at 45°C+/-5°C.

In a particular embodiment, the protease variant has a polyester degrading activity at 45°C +/-5°C at least 5% higher than the polyester degrading activity of the protease of SEQ ID N°1, preferably at least 10%, 20%, 50%, 100%, 200%, 300%, 500% or higher.

In a particular embodiment, the protease variant of the invention has an increased polyester degrading activity, compared to the protease of SEQ ID N°1, at a temperature between 10°C and 30°C, more preferably between 15°C and 30°C, even more preferably between 20°C and 30°C, even more preferably at 28°C +/-2°C. In a particular embodiment, the protease variant has a polyester degrading activity at 28°C +/-2°C at least 5% higher than the polyester degrading activity of the protease of SEQ ID N°1, preferably at least 10%, 20%, 50%, 100%, 200%, 300%, 500% or higher.

According to the inventions, the protease variant of the invention exhibits a measurable polyester degrading activity at least in a range of pH from 5 to 11, preferably in a range of pH from 7 to 10, more preferably in a range of pH from 7 to 9.5, even more preferably at both pH 7.5 and 9.

According to the inventions, the protease variant of the invention exhibits an increased polyester degrading activity compared to the protease of SEQ ID N°1 at least at a pH comprised between 7 and 9.5, preferably at least at pH 7.5 and/or pH 9.

In a particular embodiment, the protease variant of the invention has an increased polyester degrading activity, compared to the protease of SEQ ID N°1, at a temperature between 40°C and 60°C, in particular at 45°C+/-5°C and a pH between 7.5 and 9.

In another particular embodiment, the protease variant of the invention has an increased polyester degrading activity, compared to the protease of SEQ ID N°1, at a temperature between 20°C and 30°C, in particular at 28°C +/-2°C and a pH between 7.5 and 9.

### Thermostability

Advantageously, the thermostability of the variant proteases of the invention is not impaired compared to the thermostability of the parent protease of SEQ ID N°1. More advantageously, the thermostability of the variants is improved compared to the thermostability of the parent protease. Within the context of the invention, the term *"improved thermostability"* indicates an increased ability of an enzyme to resist to changes in its chemical and/or physical structure at high temperatures, and more particularly at temperature between 40°C and 90°C, such as 70°C, as compared to the protease of SEQ ID N°1. In particular, the proteases of the present invention can have an increased residual degrading activity at a temperature between 40°C and 90°C, such as 70°C, as compared to the protease of SEQ ID N°1.

The thermostability of a protein may be evaluated by the one skilled in the art, according to methods known per se in the art. For instance, thermostability can be assessed by measuring the residual protease activity and/or the residual polyester depolymerization activity (i.e., polyester degrading activity) of the enzyme after incubation at different temperatures and comparing with the residual protease activity and/or residual polyester depolymerization activity of the parent protease. The ability to perform multiple rounds of polyester's depolymerization assays at different temperatures can also be evaluated. A rapid and qualitative test may consist on the evaluation, by halo diameter measurement, of the enzyme ability to degrade a solid polyester compound dispersed in an agar plate after incubation at different temperatures. Alternatively or in addition, a Differential Scanning Fluorimetry (DSF) or Nano differential scanning fluorimetry (Nano-DSF) may be performed to assess the thermostability of a protein/enzyme by measuring the melting temperature (Tm) of said protease. Alternatively, the Tm can be assessed by analysis of the protein folding using circular dichroism. Both methods are used to quantify the change in thermal denaturation temperature of a protein and thereby to determine its melting temperature (Tm). In the context of the invention the *"melting temperature (Tm)"* of a given protein corresponds to the temperature at which half of protein population considered is denatured (unfolded or misfolded).. In the context of the invention, comparisons of Tm are performed with Tm that are measured under same conditions (e.g. pH, nature and amount of polyesters, etc.). Advantageously, the protease of the invention exhibits an equivalent melting temperature (Tm) as the Tm of the protease of SEQ ID N°1, i.e., the ΔTm between the protease variant and the protease of SEQ ID N°1 is below 0.8°C in same experimental conditions (e.g., at pH9).

Advantageously, the protease of the invention exhibits a higher or equivalent melting temperature (Tm) as compared to the protease of SEQ ID N°1. In a particular embodiment, the protease of the invention exhibits a melting temperature (Tm) above 40°C, preferably above 45°C, more preferably above 50°C.

### Nucleic acids, expression cassette, vector, host cell

It is a further object of the invention to provide a nucleic acid encoding a protease as defined above.

As used herein, the term *"nucleic acid*", *"nucleic sequence*,*" "polynucleotide"*, *"oligonucleotide"* and *"nucleotide sequence"* are used interchangeably and refer to a sequence of deoxyribonucleotides and/or ribonucleotides. The nucleic acids can be DNA (cDNA or gDNA), RNA, or a mixture thereof. It can be in single stranded form or in duplex form or a mixture thereof. It can be of recombinant, artificial and/or synthetic origin and it can comprise modified nucleotides, comprising for example a modified bond, a modified purine or pyrimidine base, or a modified sugar. The nucleic acids of the invention can be in isolated or purified form, and made, isolated and/or manipulated by techniques known per se in the art, e.g., cloning and expression of cDNA libraries, amplification, enzymatic synthesis or recombinant technology. The nucleic acids can also be synthesized in vitro by well-known chemical synthesis techniques, as described in, e.g., Belousov (1997) Nucleic Acids Res. 25:3440-3444.

The invention also encompasses nucleic acids which hybridize, under stringent conditions, to a nucleic acid encoding a protease as defined above. Preferably, such stringent conditions include incubations of hybridization filters at about 42°C for about 2.5 hours in 2 X SSC/0.1%SDS, followed by washing of the filters four times of 15 minutes in 1 X SSC/0.1% SDS at 65° C. Protocols used are described in such reference as Sambrook et al. (Molecular Cloning: a Laboratory Manual, Cold Spring Harbor Press, Cold Spring Harbor N.Y. (1988)) and Ausubel (Current Protocols in Molecular Biology (1989)).

The invention also encompasses nucleic acids encoding a protease of the invention, wherein the sequence of said nucleic acids, or a portion of said sequence at least, has been engineered using optimized codon usage.

Alternatively, the nucleic acids according to the invention may be deduced from the sequence of the protease according to the invention and codon usage may be adapted according to the host cell in which the nucleic acids shall be transcribed. These steps may be carried out according to methods well known to one skilled in the art and some of which are described in the reference manual Sambrook et al. (Sambrook et al., 2001).

Nucleic acids of the invention may further comprise additional nucleotide sequences, such as regulatory regions, i.e., promoters, enhancers, silencers, terminators, signal peptides and the like that can be used to cause or regulate expression of the polypeptide in a selected host cell or system. Alternatively, or in addition, nucleic acids of the invention may further comprise additional nucleotide sequences encoding fusion proteins, such as maltose binding protein (MBP) or glutathion S transferase (GST) that can be used to favor polypeptide expression and/or solubility.

The present invention further relates to an expression cassette comprising a nucleic acid according to the invention operably linked to one or more control sequences that direct the expression of said nucleic acid in a suitable host cell. The term *"expression cassette"* denotes a nucleic acid construct comprising a coding region, i.e. a nucleic acid of the invention, and a regulatory region, i.e. comprising one or more control sequences (e.g., transcriptional promoter and/or transcription terminator). The control sequence may include a promoter that is recognized by a host cell or an *in vitro* expression system for expression of a nucleic acid encoding a protease of the present invention. The promoter contains transcriptional control sequences that mediate the expression of the enzyme. The promoter may be any polynucleotide that shows transcriptional activity in the host cell including mutant, truncated, and hybrid promoters, and may be obtained from genes encoding extracellular or intracellular polypeptides either homologous or heterologous to the host cell. The control sequence may also be a transcription terminator, which is recognized by a host cell to terminate transcription. The terminator is operably linked to the 3'-terminus of the nucleic acid encoding the protease. Any terminator that is functional in the host cell may be used in the present invention. Typically, the expression cassette comprises, or consists of, a nucleic acid according to the invention operably linked to a transcriptional promoter and a transcription terminator.

The invention also relates to a vector comprising a nucleic acid or an expression cassette as defined above.

The term *"vector"* refers to DNA or RNA molecule used as a vehicle to transfer recombinant genetic material into a host cell. The vector may be linear or circular, single- or double-stranded DNA or RNA. The vector may be integrative or extrachromosomal, or autoreplicative. Preferably, the expression vector is a linear or circular double stranded DNA molecule. The major types of vectors are plasmids, bacteriophages, viruses, fosmids, cosmids, and artificial chromosomes. The vector itself is generally a DNA or RNA sequence that consists of an insert (a heterologous nucleic acid sequence, transgene) and a larger sequence that serves as the "backbone" of the vector. The purpose of a vector which transfers genetic information to the host is typically to isolate, multiply, or express the insert in the target cell. Vectors called expression vectors (expression constructs) are specifically adapted for the expression of the heterologous sequences in the target cell, and generally have a promoter sequence that drives expression of the heterologous sequences encoding a polypeptide. As used herein, the term *"expression vector"* means a DNA or RNA molecule that comprises an expression cassette of the invention. Generally, the regulatory elements that are present in an expression vector include a transcriptional promoter, a ribosome binding site, a terminator, and optionally present operator. Preferably, an expression vector also contains an origin of replication for autonomous replication in a host cell, a selectable marker, a limited number of useful restriction enzyme sites, and a potential for high copy number. Examples of expression vectors are cloning vectors, modified cloning vectors, specifically designed plasmids and viruses. Expression vectors providing suitable levels of polypeptide expression in different hosts are well known in the art. The choice of the vector will typically depend on the compatibility of the vector with the host cell into which the vector is to be introduced. Preferably, the expression vector is a linear or circular double stranded DNA molecule.

It is another object of the invention to provide a host cell comprising a nucleic acid, an expression cassette or a vector as described above. The present invention thus relates to the use of a nucleic acid, expression cassette or vector according to the invention to transform, transfect or transduce a host cell. The choice of the vector will typically depend on the compatibility of the vector with the host cell into which it must be introduced.

According to the invention, the host cell may be transformed, transfected or transduced in a transient or stable manner. The expression cassette or vector of the invention is introduced into a host cell so that the cassette or vector is maintained as a chromosomal integrant or as a self-replicating extra-chromosomal vector. The term "host cell" also encompasses any progeny of a parent host cell that is not identical to the parent host cell due to mutations that occur during replication. The host cell may be any cell useful in the production of a variant of the present invention, e.g., a prokaryote or a eukaryote. The prokaryotic host cell may be any Gram-positive or Gram-negative bacterium. The host cell may also be a eukaryotic cell, such as a yeast, fungal, mammalian, insect or plant cell. In a particular embodiment, the host cell is selected from a prokaryotic host cell, such as bacteria, and yeast or fungal host cell. In a particular embodiment, the host cell is selected from the group of *Escherichia coli, Bacillus, Streptomyces, Trichoderma, Aspergillus, Saccharomyces, Pichia, Thermus, Actinomadura* or *Yarrowia.* More preferably, the host cell is selected from the group of *Escherichia coli, Bacillus, Aspergillus, and Trichoderma.*

The nucleic acid, expression cassette or expression vector according to the invention may be introduced into the host cell by any method known by the skilled person, such as electroporation, conjugation, transduction, competent cell transformation, protoplast transformation, protoplast fusion, biolistic "gene gun" transformation, PEG-mediated transformation, lipid-assisted transformation or transfection, chemically mediated transfection, lithium acetate-mediated transformation, liposome-mediated transformation.

Optionally, more than one copy of a nucleic acid, cassette or vector of the present invention may be inserted into a host cell to increase production of the variant.

In a particular embodiment, the host cell is a recombinant microorganism. The invention indeed allows the engineering of microorganisms with improved capacity to degrade polyester containing material. For instance, the sequence of the invention may be used to complement a wild type strain of a fungus or bacterium already known as able to degrade polyester, in order to improve and/or increase the strain capacity.

### Production of protease variants

It is another object of the invention to provide a method of producing a protease variant of the invention, comprising expressing a nucleic acid encoding the protease and optionally recovering the protease.

In particular, the present invention relates to *in vitro* methods of producing a protease of the present invention comprising (a) contacting a nucleic acid, cassette or vector of the invention with an *in vitro* expression system; and (b) recovering the protease produced. *In vitro* expression systems are well-known by the person skilled in the art and are commercially available.

Preferably, the method of production comprises
(a) culturing a host cell that comprises a nucleic acid encoding a protease of the invention under conditions suitable to express the nucleic acid; and optionally
(b) recovering said protease from the cell culture.

Advantageously, the host cell is a recombinant *Bacillus,* recombinant *E. coli,* recombinant *Aspergillus,* recombinant *Trichoderma,* recombinant *Streptomyces,* recombinant *Saccharomyces,* recombinant *Pichia,* recombinant *Thermus,* recombinant *Actinomadura* or recombinant *Yarrowia.* Preferably, the host cell is selected from recombinant *Bacillus,* recombinant E. *coli,* recombinant *Aspergillus,* recombinant *Trichoderma.*

The host cells are cultivated in a nutrient medium suitable for production of polypeptides, using methods known in the art. For example, the cell may be cultivated by shake flask cultivation, or small-scale or large-scale fermentation (including continuous, batch, fed- batch, or solid state fermentations) in laboratory or industrial fermentors performed in a suitable medium and under conditions allowing the enzyme to be expressed and/or isolated. The cultivation takes place in a suitable nutrient medium, from commercial suppliers or prepared according to published compositions (e.g., in catalogues of the American Type Culture Collection) or any other culture medium suitable for cell growth.

If the protease is excreted into the nutrient medium, the protease can be recovered directly from the culture supernatant. Conversely, the protease can be recovered from cell lysates or after permeabilization. The protease may be recovered using any method known in the art. For example, the protease may be recovered from the nutrient medium by conventional procedures including, but not limited to, collection, centrifugation, filtration, extraction, spray-drying, evaporation, or precipitation. Optionally, the protease may be partially or totally purified by a variety of procedures known in the art including, but not limited to, thermal chock, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction to obtain substantially pure polypeptides.

The protease may be used as such, in purified form, either alone or in combination with additional enzymes, to catalyze enzymatic reactions involved in the degradation and/or recycling of polyester(s) and/or polyester containing materials, such as plastic products containing polyester. The protease may be in soluble form, or on solid phase. In particular, it may be bound to cell membranes or lipid vesicles, or to synthetic supports such as glass, plastic, polymers, filter, membranes, e.g., in the form of beads, columns, plates and the like.

### Composition

It is a further object of the invention to provide a composition comprising a protease or a host cell of the invention or extract thereof. In the context of the invention, the term *"composition"* encompasses any kind of compositions comprising a protease or host cell of the invention. In a particular embodiment, the protease is in isolated or at least partially purified form.

The composition may be liquid or dry, for instance in the form of a powder. In some embodiments, the composition is a lyophilisate. For instance, the composition may comprise the protease and/or host cells encoding the protease of the invention or extract thereof containing said protease, and optionally additives such as excipients and/or reagents etc. Appropriate excipients encompass buffers commonly used in biochemistry, agents for adjusting pH, preservatives such as sodium benzoate, sodium sorbate or sodium ascorbate, conservatives, protective or stabilizing agents such as starch, dextrin, arabic gum, salts, sugars e.g. sorbitol, trehalose or lactose, glycerol, polyethyleneglycol, polyethene glycol, polypropylene glycol, propylene glycol, divalent ions such as calcium, sequestering agent such as EDTA, reducing agents, amino acids, a carrier such as a solvent or an aqueous solution, and the like. The composition of the invention may be obtained by mixing the protease with one or several excipients.

The composition of the invention may comprise from 0.1% to 99.9%, preferably from 0.1% to 50%, more preferably from 0.1% to 30%, even more preferably from 0.1% to 5% by weight of the protease of the invention and from 0.1% to 99.9%, preferably from 50% to 99.9%, more preferably from 70% to 99.9%, even more preferably from 95% to 99.9% by weight of excipient(s). A preferred composition comprises between 0.1 and 5% by weight of the protease of the invention. In an embodiment, the composition of the invention may comprise from 0.1% to 40%, more preferably from 1% to 30%, even more preferably from 5% to 25% by weight of the protease of the invention and from 60% to 99.9%, preferably from 70% to 99%, more preferably from 75% to 95% by weight of excipient(s).

In a particular embodiment, the composition may further comprise additional polypeptide(s) exhibiting an enzymatic activity. The amounts of protease of the invention will be easily adapted by those skilled in the art depending e.g., on the nature of the polyester and/or polyester containing material to degrade and/or the additional enzymes/polypeptides contained in the composition.

In a particular embodiment, the protease of the invention is solubilized in an aqueous medium together with one or several additives such as excipients, especially excipients which are able to stabilize or protect the polypeptide from degradation. For instance, the protease of the invention may be solubilized in water, eventually with additional components, such as glycerol, sorbitol, dextrin, starch, glycol such as propanediol, salt, etc. The resulting mixture may then be dried so as to obtain a powder. Methods for drying such mixture are well known to the one skilled in the art and include, without limitation, lyophilisation, freeze-drying, spray-drying, supercritical drying, down-draught evaporation, thin-layer evaporation, centrifugal evaporation, conveyer drying, fluidized bed drying, drum drying or any combination thereof.

In a further particular embodiment, the composition of the invention comprises at least one host cell expressing a protease of the invention, or an extract thereof comprising said protease. An *"extract of a cell*" designates any fraction obtained from a cell, such as cell supernatant, cell debris, cell walls, DNA extract, enzymes or enzyme preparation or any preparation derived from cells by chemical, physical and/or enzymatic treatment, which is essentially free of living cells. Preferred extracts are enzymatically-active extracts. The composition of the invention may comprise one or several host cells of the invention or extract thereof containing the protease of the invention, and optionally one or several additional cells.

In a particular embodiment, the composition consists or comprises a lyophilized culture medium of a recombinant microorganism expressing and/or excreting a protease of the invention. In a particular embodiment, the powder comprises the protease of the invention and a stabilizing/solubilizing amount of glycerol, sorbitol or dextrin, such as maltodextrine and/or cyclodextrine, starch, Arabic gum, glycol such as propanediol, and/or salt.

### Uses of the proteases

It is a further object of the invention to provide methods using a protease of the invention for degrading and/or recycling in aerobic or anaerobic conditions polyester and/or polyester containing material, as plastic products made of or containing polyesters and/or producing biodegradable plastic products containing polyester. The proteases of the invention are particularly useful for producing biodegradable plastic products containing PLA and/or for degrading PLA and a plastic product comprising PLA.

It is therefore an object of the invention to use a protease of the invention, or corresponding host cell or extract thereof containing such protease, or composition, for the enzymatic degradation of a polyester or a polyester containing material, such as PLA or a PLA containing material.

It is another object of the invention to provide a method for degrading a polyester or a plastic product containing at least one polyester, wherein the polyester or the plastic product is contacted with a protease or host cell or composition of the invention. Advantageously, polyester(s) and/or polyester(s) of the polyester containing material is (are) depolymerized up to monomers and/or oligomers. In an embodiment of the method of degradation, at least one polyester is degraded to yield repolymerizable monomers and/or oligomers, which are advantageously retrieved in order to be used.

In a preferred embodiment of the method of degradation, the polyester is PLA and at least PLA is degraded to yield repolymerizable monomers and/or oligomers of lactic acid (LA), which are advantageously retrieved in order to be used for instance to produce new polymers of PLA.

In an embodiment, polyester(s) and/or polyester(s) of the polyester containing material is (are) fully degraded.

In a particular embodiment, the polyester is selected from polylactic acid (PLA), poly(glycolic acid) (PGA), poly(lactic-co-glycolic acid) (PLGA), polytrimethylene terephthalate (PTT), polybutylene terephthalate (PBT), polyethylene isosorbide terephthalate (PEIT), polyethylene terephthalate (PET), polyhydroxyalkanoate (PHA), polybutylene succinate (PBS), polybutylene succinate adipate (PBSA), polybutylene adipate terephthalate (PBAT), polyethylene furanoate (PEF), polycaprolactone (PCL), poly(ethylene adipate) (PEA) and blends/mixtures thereof, preferably polylactic acid.

In a particular embodiment, the plastic product comprises at least one polyester selected from polylactic acid (PLA), polytrimethylene terephthalate (PTT), polybutylene terephthalate (PBT), polyethylene isosorbide terephthalate (PEIT), polyethylene terephthalate (PET), polyhydroxyalkanoate (PHA), polybutylene succinate (PBS), polybutylene succinate adipate (PBSA), polybutylene adipate terephthalate (PBAT), polyethylene furanoate (PEF), polycaprolactone (PCL), poly(ethylene adipate) (PEA) and blends/mixtures of these materials, preferably polylactic acid.

In a particular embodiment, PLA or a plastic product containing PLA is contacted with a protease or host cell of the invention and PLA is degraded to monomers and/or oligomers of lactic acid. In a preferred embodiment, monomers and/or oligomers of lactic acid are recovered for recycling, polymerizing PLA or methanisation for instance.

The invention also relates to a method of producing monomers and/or oligomers from a polyester or polyester containing material, comprising exposing a polyester or polyester containing material to a protease of the invention, or corresponding host cell or extract thereof, or composition, and optionally recovering monomers and/or oligomers. The method of the invention is particularly useful for producing monomers such as lactic acid from plastic product containing PLA.

The time required for degrading a polyester or polyester containing material may vary depending on the polyester or polyester containing material itself (i.e., nature and origin of the plastic product, its composition, shape etc.), the type and amount of protease used, as well as various process parameters (i.e., temperature, pH, additional agents, etc.). One skilled in the art may easily adapt the process parameters to the polyester containing material.

Advantageously, the degrading process is implemented at a temperature comprised between 10°C and 90°C, e.g.; between 20°C to 70°C, between 20°C and 90°C, between 30°C to 60°C, between 40°C and 80°Cpreferably at 45°C, +/-5°C. More generally, the temperature is maintained below an inactivating temperature, which corresponds to the temperature at which the protease is inactivated and/or the recombinant microorganism does no more synthesize the protease. Preferably, the temperature is maintained below the glass transition temperature (Tg) of the polyester in the polyester containing material. In this embodiment, the degrading process is implemented at a temperature below 80°C, preferably below 70°C, more preferably below 60°C, more preferably at or below 50°C, even more preferably at 45°C, +/-5°C. More particularly, the process is implemented in a continuous way, at a temperature at which the protease can be used several times and/or recycled.

Advantageously, the degrading process is implemented at a pH comprised between 5 and 11, preferably at a pH between 6 and 10, more preferably at a pH between 6.5 and 9, in particular between pH7 and pH8 or between pH7.5 and pH9. In an embodiment, the degrading process is implemented at pH 7.5 or pH 9.

In a particular embodiment, the degrading process is implemented at a temperature between 40°C and 60°C, in particular at 45°C+/-5°C and a pH between 7.5 and 9.In a particular embodiment, the polyester or polyester containing material may be pretreated prior to be contacted with the protease, in order to physically change its structure, so as to increase the surface of contact between the polyester and the enzyme.

Optionally, monomers and/or oligomers resulting from the depolymerization may be recovered, sequentially or continuously. A single type of monomers and/or oligomers or several different types of monomers and/or oligomers may be recovered, depending on the starting polyester containing material.

The recovered monomers and/or oligomers may be further purified, using all suitable purifying methods and conditioned in a repolymerizable form. Examples of purifying methods include stripping process, separation by aqueous solution, steam selective condensation, filtration and concentration of the medium after the bioprocess, separation, distillation, vacuum evaporation, extraction, electrodialysis, adsorption, ion exchange, precipitation, crystallization, concentration and acid addition dehydration and precipitation, nanofiltration, acid catalyst treatment, semi continuous mode distillation or continuous mode distillation, solvent extraction, evaporative concentration, evaporative crystallization, liquid/liquid extraction, hydrogenation, azeotropic distillation process, adsorption, column chromatography, simple vacuum distillation and microfiltration, combined or not.

The repolymerizable monomers and/or oligomers may then be used for instance to synthesize polyesters. Advantageously, polyesters of same nature are repolymerized. However, it is possible to mix the recovered monomers and/or oligomers with other monomers and/or oligomers, in order for instance to synthesize new copolymers. Alternatively, the recovered monomers may be used as chemical intermediates in order to produce new chemical compounds of interest.

It is a further object of the invention to provide a polyester or polyester containing material in which a protease of the invention and/or a recombinant microorganism expressing and/or excreting said protease and/or extract thereof containing such protease, and/or a composition of the invention is/are included. In a particular embodiment, such polyester containing material may be a plastic compound, a masterbatch composition and/or a plastic product. In the context of the invention, a "masterbatch composition" refers to a concentrated mixture of selected ingredients (e.g., active agents, additives, etc.) that can be used for introducing said ingredients into plastic compound or product in order to impart desired properties thereto. Masterbatch compositions may be solid or liquid. Preferably, masterbatch compositions of the invention contain at least 10% by weight of active ingredients, more preferably of protease or composition of the invention.

It is thus a further object of the invention to provide a plastic compound containing a protease of the invention and/or a recombinant microorganism expressing and/or excreting said protease or extract thereof containing such protease and/or a composition of the invention and at least one polyester. In a particular embodiment, the polyester is polylactic acid (PLA), preferably poly(L-lactic acid) (PLLA), poly(D-lactic acid) (PDLA) or poly(DL-lactic acid) (PDLLA). In a particular embodiment, the plastic compound may contain an additional polymer, preferably selected from polyesters such as PBAT, PCL, PET; polyolefins such as polyethylene, polypropylene or natural polymers such as starch, cellulose or flour; and blends/mixtures thereof. More particularly, the plastic compound may contain additional polymers selected from PBAT, flour or starch. In another particular embodiment, the polyester is polycaprolactone (PCL).

It is thus a further object of the invention to provide a masterbatch composition containing a protease of the invention and/or a recombinant microorganism expressing and/or excreting said protease or extract thereof containing such protease and/or a composition of the invention, and at least one polyester. In a particular embodiment, the polyester is polylactic acid (PLA), preferably poly(L-lactic acid) (PLLA), poly(D-lactic acid) (PDLA) or poly(DL-lactic acid) (PDLLA). In another particular embodiment, the polyester is preferably polycaprolactone (PCL).

In particular, the invention relates to a process for producing such polyester containing material (i.e., plastic compound, masterbatch composition or plastic product) comprising a step of mixing a polyester and a protease and/or recombinant microorganism of the invention or extract thereof containing such protease and/or a composition of the invention, able to degrade said polyester, at a temperature at which the polyester is in a partially or totally molten state so that the protease/ microorganisms are integrated into the very structure of the polyester containing material. In a particular embodiment, the process is an extrusion process.

For instance, the protease and/or the composition of the invention and the polyester may be mixed at a temperature between the glass transition temperature and the melting point of the polyester. Alternatively, the protease/ composition of the invention and the polyester may be mixed at a temperature corresponding to the melting point of said polyester, or above. In a particular embodiment, the protease/ composition and the polyester are mixed at a temperature between 40°C and 250°C, preferably between 50°C and 180°C. Alternatively, the polypeptide/ composition and the polyester are mixed at a temperature above 40°C, preferably above 50°C, even more preferably above 60°C.

In a preferred embodiment, the polyester is selected from polylactic acid (PLA), and the protease/ composition and PLA are mixed at a temperature between 60°C and 250°C, preferably between 100°C and 200°C, more preferably between 130°C and 180°C, even more preferably between 140°C and 160°C. Alternatively, the protease/ composition and PLA are mixed at a temperature above 80°C, preferably, above 100°C, even more preferably above 130°C, and below 180°C.

In another preferred embodiment, the polyester is selected from polycaprolactone (PCL), and the protease /composition and PCL are mixed at a temperature between 40°C and 100°C, preferably between 50°C and 80°C. Alternatively, the protease / composition and PCL are mixed at a temperature above 40°C, preferably, above 50°C, even more preferably above 55°C, and below 80°C.

More preferably, the mixing step is performed using extrusion, twin screw extrusion, single screw extrusion, injection-molding, casting, thermoforming, rotary molding, compression, calendering, ironing, coating, stratification, expansion, pultrusion, extrusion blow-molding, extrusion-swelling, compression-granulation, water-in-oil-in-water double emulsion evaporation, 3D printing or any techniques known by person skilled in the art.

The resulting plastic compound, masterbatch composition or plastic product integrates protease/microorganism or composition of the invention embedded in the mass of the compound, masterbatch composition or plastic product.

Advantageously, such plastic compound, masterbatch composition can be used for the production of polyester containing materials and/or plastic article that will thus include the polypeptide of the invention.

In a particular embodiment, the resulting plastic compound, masterbatch composition or plastic article is a biodegradable plastic compound, masterbatch composition or plastic article complying with at least one of the relevant standards and/or labels known by the person skilled in the art, such as standard EN 13432, standard ASTM D6400, OK Biodegradation Soil (Label Vinçotte), OK Biodegradation Water (Label Vinçotte), OK Compost (Label Vinçotte), OK Home Compost (Label Vinçotte).

Advantageously, the degrading process of the polyester containing material (i.e., plastic compound, masterbatch composition or plastic product) is implemented at a temperature comprised between 10°C and 50°C, preferably between 15°C and 40°C, more preferably between 20°C and 30°C, more preferably at 28°C, +/- 2°C.

Alternatively, the degrading process of the polyester containing material (i.e., plastic compound, masterbatch composition or plastic product) is implemented at a temperature comprised between 50°C and 60°C, more preferably at 55°C, +/- 2°C.

Additionally to the use of the protease of the invention for degrading polyester and polyester containing material, the protease of the present invention may be used in detergent, food, animal feed and pharmaceutical applications.

More particularly, a protease of the invention may be used as a component of a detergent composition.

Detergent compositions include, without limitation, hand or machine laundry detergent compositions, such as laundry additive composition suitable for pre-treatment of stained fabrics and rinse added fabric softener composition, detergent composition for use in general household hard surface cleaning operations, detergent compositions for hand or machine dishwashing operations.

In a particular embodiment, a protease of the invention may be used as a detergent additive. The invention thus provides detergent compositions comprising a protease of the invention.

The present invention is also directed to methods for using a protease of the invention in animal feed, as well as to feed compositions and feed additives comprising a protease of the invention. The terms "feed" and "feed composition" refer to any compound, preparation, mixture, or composition suitable for, or intended for intake by an animal. In another particular embodiment, the protease of the invention is used to hydrolyze proteins, and to produce hydrolysates comprising peptides. Such hydrolysates may be used as feed composition or feed additives.

The invention also relates to a method of surface hydrolysis or surface functionalization of a polyester containing material, comprising exposing a polyester containing material to a protease of the invention, or corresponding recombinant cell or extract thereof, or composition. The method of the invention is particularly useful for increasing hydrophilicity, or water absorbency, of a polyester material. Such increased hydrophilicity may have particular interest in textiles production, electronics and biomedical applications.

### EXAMPLES

### Example 1 -Construction, expression and purification of proteases

### 1.1 Construction

The gene encoding for the non-matured parent protease (SEQ ID N°2 + SEQ ID N°1) is cloned in the plasmid pET26b+ (EMD Millipore, Billerica, Massachusetts, USA), in frame with sequences encoding PelB signal peptide (MKYLLPTAAAGLLLLAAQPAMA - SEQ ID N°3) upstream of the gene and a 6xhistidine tag (LEHHHHHH - SEQ ID N°4) downstream of the gene. Competent *E*. *coli* One Shot^{®} BL21 DE3 (Life technologies, Carlsbad, California, USA) is transformed with the constructed plasmid. The obtained strain expresses the parent protease with a PelB leader sequence at the N-terminal and a 6X histidine Tag at the C-terminal of the protein. QuikChange II Site-Directed Mutagenesis kit is used according to the recommendations of the supplier to construct the variants (Santa Clara, California, USA).

### 1.2 Expression and purification of the proteases

Recombinant expression of the strains expressing the parent protease and variants thereof is realized in ZYM auto-inducible medium (Studier, 2005) for 23 h at 21°C; or in 100 mL TB medium at 37°C during 4 hours then at 21°C after IPTG addition at final concentration of 1 mM (Tartoff K. D. and Hobbs C. A., 1987, Improved Media for Growing Plasmid and Cosmid Clones, Bethesda Res. Lab. Focus, 9:12.). Cultures are stopped by centrifugation (8000 rpm, 20 minutes at 10°C) in an Avanti J-26 XP centrifuge (Beckman Coulter, Brea, USA). Cells are frozen at -80 °C during at least 2.5 hours and then suspended in 25 mL of Tris HCl buffer (Tris 0.1 M, pH 9). Lysonase^{™} Bioprocessing Reagent (EMD Millipore) can be used to lysate the cells, according to supplier's recommendation or sonication of the cell suspension during 2 minutes with 30% of amplitude (15s ON and 45s OFF cycles) using FB 705 sonicator (Fisherbrand, Illkirch, France). Cell suspension is centrifuged during 30 minutes at 11000 rpm and at 10°C. The soluble fraction is collected and submitted to cobalt affinity chromatography using Talon^{®} Metal Affinity resin (Clontech, CA, USA). Protein is eluted with 100mM imidazole in 20mM Tris-HCl, 300mM NaCl, pH 8.0. Imidazole is removed from purified extracts after a dialysis step or a desalting step with PD10 column (Sigma-Aldrich, GE17-0851-01) against Tris HCl buffer supplemented or not with calcium chloride (Tris 0.1 M, 0 or 5 mM CaCl2, pH 7.5 or pH 9 regulated at 45°C) and stored at +4°C or -20°C. The quality of the purification is assessed on SDS-PAGE by adding PMSF in excess to the protease solution, or after TCA precipitation, the expected protein size being around 29kDa. Purified protein concentrations were determined based on the calculated molar extinction coefficient (Gasteiger et al., 2005) after absorbance measurement at 280 nm using NanoDrop^{™} 2000 (ThermoFisher Scientific).

### Example 2 - Evaluation of the degrading activity of the proteases

### 2.1 Screening on dispersed nanoparticles of PLA agarose

450 mg of PLA powder (PLLA 001 - Natureplast) was dissolved in 15 mL dichloromethane (DCM) and vortexed. The addition of 90 mL of 0.1 M Tris-HCl pH 9.0 was followed by a sonication step (Fisher Scientific^{™} Model 705 Sonic Dismembrator, at 30 % of maximum power) and the DCM was further evaporated at 50°C under stirring. The resulting solution of 0.5 % (w/v) PLA emulsion was filtered (20-25 µm) to remove aggregates and undissolved large particles. Finally, 12 mL of the 0,5% PLA emulsion were mixed with 3 mL of 1 M Tris-HCl buffer pH 9 (adjusted at 45°C) and 15 mL of agarose 2 % (w/v) to obtain dispersed nanoparticles of PLA agarose plate. An aliquot of 20 µL of cell extract was deposited in a well formed in dispersed nanoparticles of PLA agarose omnitray plate. Omnitrays were then incubated at 45°C for enzymatic activity producing clear zones (also named "halos") of PLA depolymerization around the wells containing enzyme preparation. The diameters of the halos formed by the parent protease and variants of the invention were measured. The score for each variant of the invention corresponded to the relative halo diameter (in %) compared to the mean diameter of the halo formed by the parent protease performed in duplicate on the same microplate.

**Table 1: Relative degrading activity of protease variants as compared to the parent protease (SEQ ID N°1) at pH 9.0 (regulated at 45°C).**

| Variants of the invention | Relative halo diameter at pH 9.0 compared to SEQ ID N°1 (%) |
|---|---|
| V1 : S101A | 189 |
| V2 : S101G | 130 |
| V3 : S101H | 127 |
| V4 : S101I | 130 |
| V5 : S101K | 160 |
| V6: S101N | 158 |
| V7: S101Q | 127 |
| V8 : S101R | 122 |
| V9 : S101T | 138 |
| V10 : S101V | 175 |
| V11 : S103A | 135 |
| V12 : S103F | 152 |
| V13 : S103H | 117 |
| V14 : S103I | 139 |
| V15 : S103K | 117 |
| V16 : S103M | 114 |
| V17: S103N | 111 |
| V18 : S103Q | 141 |
| V19 : S103R | 136 |
| V20 : S103T | 150 |
| V21 : S103V | 153 |
| V22 : S103W | 122 |
| V23 : S103Y | 118 |
| V24 : T106V | 116 |
| V25 : G131L | 107 |
| V26 : G131T | 110 |
| V27 : G131V | 114 |
| V28 : I217L | 105 |

The variants listed in Table 1 have the exact amino acid sequence of SEQ ID N°1 except the substitutions listed in Table 1.

### 2.2 Liquid PLA depolvmerase activity assay

The specific degrading activities of parent protease and variants thereof was determined using PLA hydrolysis in liquid medium. 50 mg of a 500µm PLA powder (PLLA 001 - Natureplast) were weighed and introduced in dialysis tubing. The variants have the amino acid sequence as set forth in SEQ ID N°1, except specific substitutions, as listed. The enzymatic sample (1.5 mL of protease preparation containing a fixed concentration of enzyme of 0.17 µM in order to measure the accurate specific activity) was then added in the dialysis tubing before closing it and this latter was introduced in a glass bottle containing 25 mL of 0.1 M Tris-HCl buffer pH 9 or pH 7.5 (regulated at 45°C). The parent protease (SEQ ID N°1) was used as a control.

The depolymerization started by incubating each sample at 45°C and 170 rpm in a Max Q 4450 incubator (Thermo Fisher Scientific, Inc. Waltham, MA, USA).

Initial rate of the depolymerization reaction in g of lactic acid and/or dimers of lactic acid generated / g of enzyme / hour was determined by samplings performed at different times during the first 24 hours and analyzed by Ultra High Performance Liquid Chromatography (UHPLC). To simplify the analysis, 3µl of NaOH 13M can be added to 200µl samples to ensure complete hydrolysis of dimers into lactic acid after 15 minutes at 95°C. If necessary, samples were diluted in 0.1 M Tris-HCl buffer pH 9 or pH 7.5 (regulated at 45°C). After filtration on 0.45 µm syringe filter, samples were loaded on UHPLC to monitor the liberation of lactic acid and dimers of lactic acid. Chromatography system used was an Ultimate 3000 UHPLC system (Thermo Fisher Scientific, Inc. Waltham, MA, USA) including a pump module, an autosampler, a column oven thermostated at 50°C, and a UV detector at 210 nm. The column used was an Aminex HPX-87H (300 mm × 7.8 mm), equipped with precolumn, (Supelco, Bellefonte, USA). Lactic acid and dimers of lactic acid were separated using a mobile phase H₂SO₄ 5 mM, at a flow rate of 0.5 mL.min⁻¹. Injection is 20 µL of sample. Lactic acid and dimers of lactic acid were measured according to standard curves prepared from commercial lactic acid (Sigma-Aldrich L1750-10G) and in house synthetized dimers of lactic acid in the same conditions than samples. The specific degrading activity of PLA hydrolysis (g of equivalent lactic acid, i.e., g of lactic acid and of dimer of lactic acid/hour/g of enzyme) was determined in the linear part of the hydrolysis curve.

Relative specific degrading activity of protease variants of the invention are shown in Table 2 below. The specific degrading activity of the protease of SEQ ID N°1 is used as a reference and considered as 100% specific degrading activity. The specific degrading activity was measured at 45°C and pH 9.

**Table 2: Relative specific degrading activity of protease variants of the invention as compared to the parent protease (SEQ ID N°1) at pH 9.0 (regulated at 45°C).**

| Variants of the invention | Relative SA at pH 9.0 compared to compared to SEQ ID N°1 (%) |
|---|---|
| V1 : S101A | 112 |
| V10 : S101V | 141 |
| V29 : G102A | 125 |
| V11 : S103A | 131 |
| V12 : S103F | 171 |
| V14 : S103I | 169 |
| V21 : S103V | 133 |
| V22 : S103W | 174 |
| V25 : G131L | 121 |
| V26 : G131T | 138 |
| V30 : S101F + S103F + T106I | 151 |
| V31 : S101F + S103F + T106L | 110 |

The variants listed in Table 2 have the exact amino acid sequence of SEQ ID N°1 except the substitutions listed in Table 2.

Relative specific degrading activity of protease variants of the invention at 45°C are shown in Table 3 below. The specific degrading activity of the protease of SEQ ID N°1 is used as a reference and considered as 100% specific degrading activity. The specific degrading activity was measured at 45°C and pH 7.5.

**Table 3: Relative specific degrading activity of the protease variants of the invention as compared to parent protease (SEQ ID N°1) at pH 7.5 (regulated at 45°C).**

| Variants of the invention | Relative SA at pH 7.5 compared to SEQ ID N°1 (%) |
|---|---|
| V10 : S101V | 207 |
| V29 : G102A | 151 |
| V12 : S103F | 226 |
| V14 : S103I | 186 |
| V22 : S103W | 241 |
| V30 : S101F + S103F + T106I | 389 |
| V31 : S101F + S103F + T106L | 313 |

The variants listed in Table 3 have the exact amino acid sequence of SEQ ID N°1 except the substitutions listed in Table 3.

Relative specific degrading activity of protease variants of the invention at 28°C are shown in Table 4 below. The specific degrading activity of the protease of SEQ ID N°1 is used as a reference and considered as 100% specific degrading activity. The specific degrading activity was measured at 28°C and both pH 7.5 and pH 9.

**Table 4: Relative specific degrading activity of the protease variants of the invention as compared to parent protease (SEQ ID N°1) at pH 7.5 (regulated at 45°C).**

| Variants of the invention | Relative SA at 28°C compared to SEQ ID N°1 (%) | |
|---|---|---|
| | pH 7.5 | pH 9 |
| V12 : S103F | 582 | 125 |

The variants listed in Table 4 have the exact amino acid sequence of SEQ ID N°1 except the substitutions listed in Table 4.

### Example 3 - Evaluation of the thermostability of protease of the invention

The thermostability of proteases of the invention has been determined and compared to the thermostability of the protease of SEQ ID N°1 to confirm that these substitutions have no negative impact on thermostability, i.e the delta Tm (ΔTm) compared to the parent protease of SEQ ID N°1 is below 0.8°C in same experimental conditions.

### Nano differential scanning fluorimetry (Nano-DSF)

The nanoDSF uses the intrinsic fluorescence of protein (tryptophan or tyrosin) to monitor their unfolding and obtain a *Tₘ* value. Protein samples were prepared at a concentration between 8 and 10 µM and stored in buffer consisting of 0.1 M Tris-HCl 9.0 (adjusted to pH 9 at 45°C). 10 µL of protein solutions were loaded onto a dedicated capillary sealed before loading to the instrument. NanoDSF experiments were then carried out using a Prometheus NT. Plex system **(NanoTemper Technologies,** Germany) set for detection of the 330 and 350 nm fluorescence. The samples were heated from 15 to 110°C at the rate of 1°C/min and laser power was 20 or 50%. *Tₘ* were determined from the peak(s) of the derivative of the ratio of fluorescence at 330/350 nm using the Prometheus software.

The Tm value of the parent protease of SEQ ID N°1 and protease variants were evaluated according to the protocol above at pH 9. Thermostabilities of protease variants of the invention compared to the protease of SEQ ID N°1 are shown in Table 5 below, expressed in ΔTm values.

**Table 5: ΔTm values of the proteases of the invention as compared to parent protease (SEQ ID N°1) at pH 9.**

| Variants of the invention | | *ΔTₘ*(°C) |
|---|---|---|
| | V12 : S103F | 0 |
| | V22 : S103W | -0.7 |
| | V30 : S101F + S103F + T106I | 0.9 |
| | V31 : S101F + S103F + T106L | 0.5 |

The variants listed in Table 5 have the exact amino acid sequence of SEQ ID N°1 except the substitutions listed in Table 5.

## Claims

1. A protease which comprises an amino acid sequence which (i) has at least 90%, 95%, 96%, 97%, 98% or 99% identity to the full length amino acid sequence set forth in SEQ ID N°1, and (ii) has at least one amino acid substitution selected from the group consisting in S101A/V/G/H/I/K/R/T/N/Q, G102A, S103A/F/I/V/W/H/K/M/Q/R/T/N/Y, T106V, G131L/T/V and I217L wherein the positions are numbered by reference to the amino acid sequence set forth in SEQ ID N°1, (iii) has a polyester degrading activity, and (iv) exhibits an increased polyester degrading activity compared to the protease of SEQ ID N°1.

2. The protease according to claim 1, wherein said protease comprises at least one substitution selected from S101A/V, G102A, S103A/F/I/V/W and G131L/T, preferably selected from S101V, S103A/F/I/V/W and G131T, more preferably selected from S103F/I/W even more preferably at least the substitution S103F.

3. The protease according to claim 1, wherein said protease comprises at least one amino acid substitution selected from the group consisting in G102A, S103A/F/I/VAV/H/K/M/Q/R/T/N/Y, G131L/T/V and I217L, preferably selected from G102A, S103A/F/I/V/W and G131L/T, more preferably selected from S103F/I/W and further comprises at least one substitution selected from S101F/L/M/W/Y and T106I/L, preferably at least one substitution selected from S101F and T106I/L.

4. The protease according to claim 3, wherein said protease comprises a combination of substitutions selected from S101F/L/M/W/Y + T106I/L.

5. The protease according to claim 4, wherein said protease comprises at least a combination of substitutions selected from S101F + S103F + T106I and S101F + S103F + T106L.

6. The protease according to claim 1, wherein said protease comprises at least one amino acid substitution selected from the group consisting in G102A, G131L/T/V and I217L and further comprises at least a combination of substitutions selected from S101F/L/M/W/Y + S103L + T106I/L, preferably selected from S101F + S103L + T106I and S101F + S103L + T106L.

7. The protease according to claim 1, wherein said protease comprises a single substitution selected from S101A/V/G/H/I/K/R/T/N/Q, G102A, S103A/F/I/V/W/H/K/M/Q/R/T/N/Y, T106V, G131L/T/V and I217L, preferably selected from S101A/V, G102A, S103A/F/I/V/W and G131L/T, more preferably selected from S101V, S103A/F/I/V/W and G131T, even more preferably selected from S103F/I/W, preferably the substitution S103F.

8. The protease according to any one of the previous claims, wherein said protease comprises at least one amino acid residue selected from D40, H71, S221, C68, C100, C164 or C195 as in the parent protease of SEQ ID N°1, preferably at least the combination of amino acid residues selected from D40 + H71 + S221 + C68 + C100, D40 + H71 + S221 + C164 + C195 and D40 + H71 + S221 + C68 + C100 + C164 + C195 as in the parent protease.

9. The protease according to any one of the previous claims, wherein said protease exhibits an increased polyester degrading activity compared to the protease of SEQ ID N°1 at temperatures between 40°C to 60°C, preferably at 45°C, +/- 5°C and/or at temperatures between 20°C and 30°C, preferably at 28°C +/-2°C.

10. The protease according to any one of the previous claims, wherein said protease exhibits an increased polyester degrading activity compared to the protease of SEQ ID N°1 at a pH comprised between 7 and 9.5, in particular at pH 7.5 and/or at pH 9.

11. A method of degrading a plastic product containing at least one polyester comprising
- contacting the plastic product with a protease according to any one of claims 1 to 10 or an host cell comprising and expressing a nucleic acid encoding the protease according to any one of claims 1 to 10 or a composition comprising the protease according to any one of claims 1 to 10; and, optionally
- recovering monomers and/or oligomers.

12. The method of claim 11, wherein the polyester is selected from the group consisting in polylactic acid (PLA), polytrimethylene terephthalate (PTT), polybutylene terephthalate (PBT), polyethylene isosorbide terephthalate (PEIT), polyethylene terephthalate (PET) polyhydroxyalkanoate (PHA), polybutylene succinate (PBS), polybutylene succinate adipate (PBSA), polybutylene adipate terephthalate (PBAT), polyethylene furanoate (PEF), polycaprolactone (PCL), polyethylene adipate) (PEA), poly(glycolic acid) (PGA), poly(lactic-co-glycolic acid) (PLGA) and blends/mixtures thereof, preferably polylactic acid (PLA).

13. A plastic compound containing (i) at least one polyester, preferably at least polylactic acid and (ii) a protease of any one of claims 1 to 10 or a host cell comprising and expressing a nucleic acid encoding the protease according to any one of claims 1 to 10 or extract thereof comprising said protease or a composition comprising the protease according to any one of claims 1 to 10, wherein the protease is advantageously able to degrade at least one polyester of the plastic compound.

14. A process for producing the plastic compound of claim 13, wherein at least one polyester and a protease of any one of claims 1 to 10 or a host cell comprising and expressing a nucleic acid encoding the protease according to any one of claims 1 to 10 or extract thereof comprising said protease or a composition comprising the protease according to any one of claims 1 to 10, are mixed at a temperature at which the polyester is in a partially or totally molten state, preferably by extrusion.

15. Use of a protease according to any one of claims 1 to 10 or host cell comprising and expressing a nucleic acid encoding the protease according to any one of claims 1 to 10 or a composition comprising the protease according to any one of claims 1 to 10, for degrading a polyester containing material, preferably for degrading a polylactic acid containing material.
